# EUROPEAN PATENT APPLICATION

(11) **EP 4 840 399 A1**
(43) Date of publication of application: **18.11.2026**
(21) Application number: 25739127.6
(22) Date of filing: 10.01.2025
(51) Int. Cl.: A61B 5/293, A61B 5/37, A61B 5/00, A61N 1/36, A61N 1/05, A61N 1/375

(54) **STRUCTURE FOR BRAIN SIGNAL MEASUREMENT AND ELECTRICAL STIMULATION, AND SYSTEM INCLUDING SAME**

(30) Priority: 11.01.2024 KR 20240005025
(71) Applicant: Gbrain Inc., Incheon 21984 (KR)
(72) Inventor: YANG, Sunggu, Incheon 22018 (KR); LEE, Sangwon, Incheon 21994 (KR); KIM, Jejung, Incheon 22228 (KR); SHIN, Jae Hoon, Incheon 21931 (KR)
(74) Representative: Patrade A/S
(86) International application number: PCT/KR2025/000607
(87) International publication number: WO 2025/150967

(57) **Abstract**

The present invention relates to a structure for signal measurement and electrical stimulation comprising a sensor unit that measures signals generated in a brain or delivers external stimuli to the brain, in which the sensor unit comprises a pad portion having a flexible property; and a rib structure portion disposed on the pad portion to increase resilience of the pad portion.

## Description

### TECHNICAL FIELD

The present invention relates to a structure for brain signal measurement and electrical stimulation and a system comprising the same, and relates to a brain neural signal measurement and electrical stimulation structure comprising a rib structure and a system comprising the same. The present invention was studied with the support of the following projects.
1. Alchemist project ([Project identification number] 1415169915 / [Project number] 20012355 / [Ministry name] Ministry of Trade, Industry, and Energy / [Project managing (specialized) institution name] Korea Planning & Evaluation Institute of Industrial Technology / [Research program name] Industrial technology alchemist project / [Research project name] Development of a totally implantable closed-loop Brain to X for voice communication / [Contribution ratio] 100% / [Project performing agency name] Gbrain, Inc. / [Research period] 2020. 09. 01 ~ 2026. 12. 31(total research period))
2. Material and component technology development program ([Project identification number] 2410002762 / [Project number] 00449917 / [Ministry name] Ministry of Trade, Industry, and Energy / [Project managing (specialized) institution name] Korea Planning & Evaluation Institute of Industrial Technology / [Research program name] Material and component technology development (R&D) / [Research project name] Wireless Closed-Loop Brain Neural Stimulator for Diagnosis/Treatment of Parkinson's Disease / [Contribution ratio] 100% / [Project performing agency name] Gbrain, Inc. / [Research period] 2024. 07. 01~2026. 12. 31)

### BACKGROUND ART

In recent years, various brain diseases such as epilepsy, Parkinson's disease, Alzheimer's disease, and stroke have been continuously increasing. As a result, technologies for effectively diagnosing and treating severe brain diseases have become an important part of the medical system. One such development is a neural recording system capable of monitoring electrocorticography (ECoG) signals over a wide area of the cortical surface. Such electrocorticography (ECoG) systems have been proven to be important diagnostic and therapeutic tools for patients suffering from severe epileptic seizures.

However, in order to accurately locate a seizure focus, surgical procedures such as craniotomy and dural incision over a wide cortical area are often required in order to insert ECoG electrodes having large dimensions into the exposed cortex. Although such procedures are necessary, they often result in significant postoperative symptoms including cerebral edema, cerebral hemorrhage, and resulting changes in intracranial pressure (ICP) and temperature (ICT).

Therefore, intracranial pressure (ICP) and temperature (ICT) monitoring technologies are frequently used to diagnose such symptoms. However, such sensors generally have bulky designs that require intracranial access, thereby potentially limiting patient mobility and impairing brain function. In addition, since separate devices are required to monitor different biological signals, there is a problem in that the surgical and postoperative processes become more complicated.

Accordingly, in the art, continuous and active research and development have been conducted in order to solve the conventional problems described above and to develop more effective methods.

### DISCLOSURE

### TECHNICAL PROBLEM

The present invention has been made in order to solve the problems of the conventional art as described above and to expand the scope of application. An object of the present invention is to provide a structure for brain signal measurement and electrical stimulation and a system comprising the same, which can simplify surgical and postoperative processes by minimizing damage during insertion of the structure into the brain while minimizing opening of the skull.

### TECHNICAL SOLUTION

The present invention relates to a structure for signal measurement and electrical stimulation comprising a sensor unit that measures signals generated in a brain or delivers external stimuli to the brain, in which the sensor unit comprises a pad portion having a flexible property; and a rib structure portion disposed on the pad portion to increase resilience of the pad portion.

Specifically, the rib structure portion may be disposed in rod shapes on an upper side of the pad portion, and the rod shapes may be formed to intersect with each other.

Specifically, the pad portion may have a radial mesh structure.

Specifically, the pad portion, may comprise a first mesh portion in which a plurality of rod shapes extending in a radial direction are spaced apart from each other in a circumferential direction; and a second mesh portion formed to intersect with the rod shapes of the first mesh portion, and in which a plurality of circular shapes forming concentric circles are spaced apart from each other in a radial direction.

Specifically, an electrode or a sensor may be installed at portions where the first mesh portion and the second mesh portion intersect.

Specifically, in the second mesh portion, a support structure may be formed on a circular shape formed at the outermost portion, and the support structure may be formed in plural and spaced apart from each other in a circumferential direction on the circular shape.

Specifically, the electrode may be composed of any one among gold, platinum-iridium alloy, graphene, carbon nanotubes, fullerene, graphite or diamond, or a composite thereof.

Specifically, the electrode, may be installed at a portion at which the rib structure portion is disposed on the first mesh portion.

Specifically, the electrode, may measure signals generated in a brain or deliver external stimulation to the brain, and the sensor may comprise a pressure sensor measuring intracranial pressure (ICP) or a temperature sensor measuring intracranial temperature (ICT).

In addition, the present invention relates to a structure for signal measurement and electrical stimulation comprising a sensor unit that measures signals generated in a brain or delivers external stimuli to the brain, wherein the sensor unit may comprise a pad portion having a flexible property; and a rib structure portion disposed on the pad portion to increase resilience of the pad portion, and further comprise an electrical pad portion for external connection; and a connection portion connecting the electrical pad portion and the sensor unit.

### ADVANTAGEOUS EFFECTS

The structure for measurement and stimulation and a system comprising the same according to one embodiment of the present invention, have effects that a rib structure is installed on a pad having a mesh structure, thereby providing structural stability to the structure, and when the pad having the flexible mesh structure is folded during insertion into the brain and then unfolded, a uniformly distributed elastic restoring force is exerted so that sufficient unfolding can be achieved.

In addition, the structure for measurement and stimulation and a system comprising the same according to one embodiment of the present invention, have a flexible radial mesh structure, thereby having inherent mechanical flexibility, and as a result, having an effect that the structure is not damaged.

### BRIEF DESCRIPTION OF THE DRAWINGS

FIG. 1 is a view illustrating the structure for measurement and stimulation according to an example of the present invention being injected into the brain.
FIG. 2 is a view illustrating, step by step, injection of the structure for measurement and stimulation according to an example of the present invention into the brain.
FIG. 3 is an exploded view of the structure for measurement and stimulation according to an example of the present invention.
FIG. 4 is a detailed view of a sensor unit of the structure for measurement and stimulation according to an example of the present invention.
FIG. 5 is a detailed view of a sensor unit of the structure for measurement and stimulation according to an example of the present invention.

### BEST MODE

The object, specific advantages, and novel features of the present invention will become more apparent from the following detailed description and preferred examples taken in conjunction with the accompanying drawings. In the present specification, when reference numerals are assigned to the components of each drawing, it should be noted that the same components are given the same reference numerals as much as possible even though they are illustrated in different drawings. In addition, in describing the present invention, when it is determined that a detailed description of related known technology may unnecessarily obscure the gist of the present invention, the detailed description thereof will be omitted.

Hereinafter, the detailed description will be described in detail with the drawings shown below.

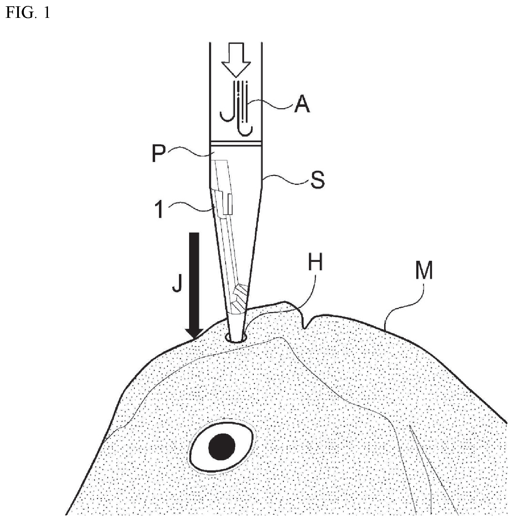

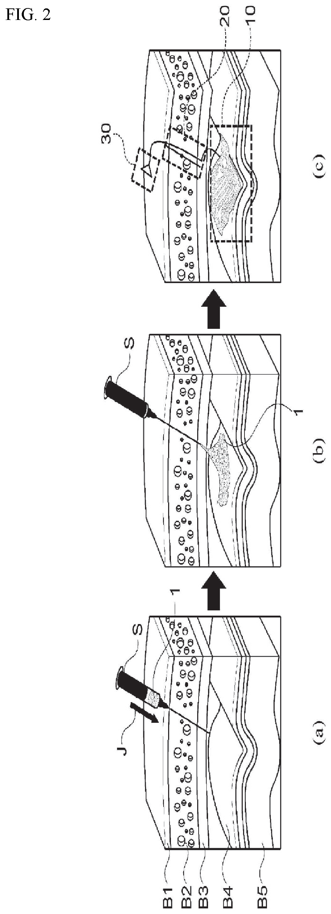

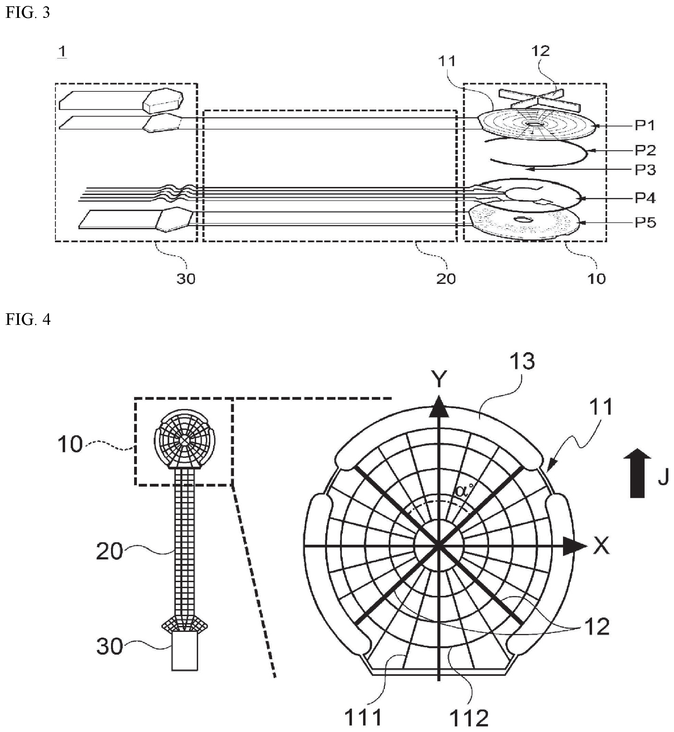

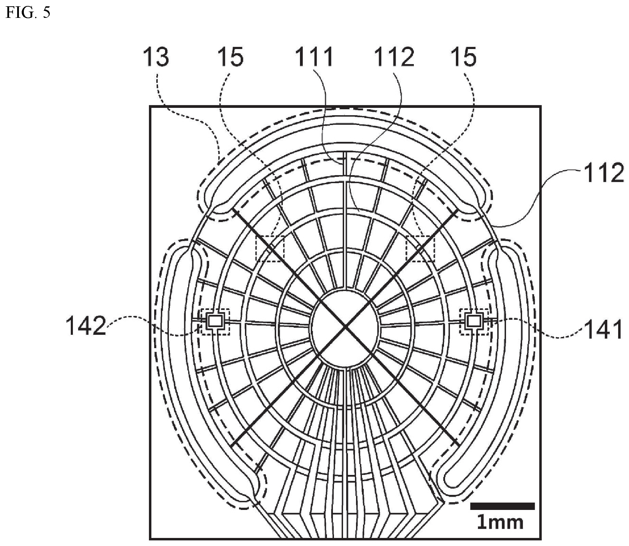

FIG. 1 is a view illustrating the structure for measurement and stimulation according to an example of the present invention being injected into the brain, and FIG. 2 is a view illustrating, step by step, injection of the structure for measurement and stimulation according to an example of the present invention into the brain, and FIG. 3 is an exploded view of the structure for measurement and stimulation according to an example of the present invention, and FIG. 4 is a detailed view of a sensor unit of the structure for measurement and stimulation according to an example of the present invention, and FIG. 5 is a detailed view of a sensor unit of the structure for measurement and stimulation according to an example of the present invention.

As shown in FIGS. 1 to 5, a structure for measurement and stimulation (1) according to an example of the present invention comprises a sensor unit (10), a connection portion (20), and an electrical pad portion (30).

Hereinafter, the structure for measurement and stimulation (1) according to the present invention having the above-described components and a system including the same will be described in detail.

In the present invention, the structure for measurement and stimulation (1) measures signals generated in a brain or delivers stimulation signals received from the outside to the brain.

The structure for measurement and stimulation (1), referring to FIG. 1, has a sensor unit (10), which is a minimally invasive multimodal array allowing to have a flexible mesh-type structure so as to be safely injected through a small hole (H) in the skull of a subject (M), and through this, accurate neural monitoring is achieved while reducing significant surgical risks of burr-hole opening and dural incision.

Referring to FIG. 1 and FIG. 2, a small hole (H) having a diameter of 1 mm~2 mm is formed in the skull, and a hole (H) of this size is sufficient for the structure for measurement and stimulation (1) of the present invention to be injected into a space (subdural space) below the dura mater (B3) between the dura mater (B3) and the arachnoid mater (B4). At this time, the structure for measurement and stimulation (1) of the present invention is folded and accommodated inside a syringe (S), and the inside of the syringe (S) is filled with a PBS (phosphate-buffered saline) solution (P) so that the structure is in a state of being immersed in the solution. The structure for measurement and stimulation (1) of the present invention may be injected into the skull of the subject (M) through the hole (H) in an injection direction (J) using air (A), thereby being injected by air pressure.

During injection, two main parameters exist. They are injection pressure and an unfolding area fraction. An appropriate injection pressure (mainly determined by bending stiffness of a device) is required for the contracted sensor unit (10) to be effectively discharged from the syringe (S), to be unfolded, and to attach to the surface of the cerebral cortex (B5). In this case, the injection pressure should be controlled within a safe range that does not damage brain cells. Since a pressure exceeding 15 kPa to 20 kPa as the injection pressure may damage brain cells, during injection into the subdural space of the present invention, the pressure is maintained below 15 kPa, and after injection, a sufficient unfolding area ratio of at least 80% may be obtained.

The structure for measurement and stimulation (1) of the present invention, as shown in FIG. 2(a), is injected in an injection direction (J) through the scalp (B1), the skull (B2), and the dura mater (B3) via a syringe (S), into a space (Subdural space) below the dura mater between the dura mater (B3) and the arachnoid mater (B4). At this time, the structure for measurement and stimulation (1) of the present invention is folded and accommodated inside the syringe (S).

Thereafter, as shown in FIG. 2(b), the structure for measurement and stimulation (1) of the present invention is dropped into the space (subdural space) below the dura mater, and is immediately unfolded so as to be suitably attached to the cerebral cortex (B5).

Thereafter, as shown in FIG. 3(c), in the structure for measurement and stimulation (1) of the present invention, the sensor unit (10) is suitably attached to the cerebral cortex (B5), a connection portion (20) is placed in a state of passing through the scalp (B1), the skull (B2), and the dura mater (B3), and the electrical pad portion (30) is placed on the scalp (B1), and at the same time, the syringe (S) is removed to the outside.

As described above, the structure for measurement and stimulation (1) of the present invention helps to significantly reduce surgical risks compared with conventional surgical methods, and has the effect that it becomes possible to simplify the entire procedure of diagnosis, treatment, and postoperative monitoring of brain diseases through multimodal ECoG signal recording, application of electrical stimulation, and monitoring of intracranial pressure (ICP) and intracranial temperature (ICT).

As shown in FIG. 3, the structure for measurement and stimulation (1) of the present invention is divided into a sensor unit (10), a connection portion (20), and an electrical pad portion (30).

A pad portion (11) and a rib structure portion (12) are formed in the sensor unit (10), and the pad portion (11) may be composed of first to fifth materials (P1 to P5). Here, the first material (P1) may be, for example, a material of SU-8, the second material (P2) may be graphene, the third material (P3) may be molybdenum disulfide (MoS₂), the fourth material (P4) may be chromium/gold (Cr/Au), and the fifth material (P5) may be polyimide. The rib structure portion (12) may be composed of, for example, a material of SU-8. The connection portion (20) and the electrical pad portion (30) may be composed of the first to fourth materials (P1~P4).

Graphene and molybdenum disulfide, which are key materials forming the structure for measurement and stimulation (1) of the present invention, have atomically thin and highly flexible characteristics. Due to these characteristics, the structure for measurement and stimulation (1) of the present invention can withstand significant deformation generated when injected through a small tip at the end of the syringe (S), and in addition, the excellent electrochemical performance and biocompatibility of graphene, and the piezoresistive property and temperature-dependent characteristic of molybdenum disulfide, can enable long-term stable and low-noise monitoring.

The sensor unit (10) measures signals generated in the brain or delivers stimulation signals received from the outside to the brain, and includes a pad portion (11) and a rib structure portion (12).

The pad portion (11) has a flexible property and may have a radial mesh structure. Conventionally, the pad portion (11) has a rectangular shape, and thus damage is easily caused due to structural stress during folding, and durability tends to be reduced. In order to overcome this, in the present invention, the pad portion (11) is formed in a circular shape rather than a rectangular shape, and a mesh form is adopted for a flexible structure.

The pad portion (11) may have a thickness of, for example, 3um~5um. Since the pad portion (11) has a mesh structure, bending stiffness is important. This bending stiffness is determined by the thickness of the pad portion (11) and the design of the rib structure portion (12) to be described later. Such design dimensions have a significant influence on mechanical dynamics such as injection pressure, mechanical stress distribution, unfolding of the substrate after discharge, and the like. For example, when the thickness of the pad portion (11) is reduced, the bending stiffness and elastic restoring force decrease, thereby reducing the injection pressure and the unfolding area ratio. Therefore, the thickness of the pad portion (11) and the rib structure portion (12) should be adjusted to optimize the injection pressure and the unfolding area ratio. When the thickness of the pad portion (11) is less than 3um, although the injection pressure condition for safe injection can be satisfied, a problem arises in that the elastic restoring force becomes low and a low unfolding area ratio of less than 34.6% is obtained. On the other hand, when the thickness of the pad portion (11) is 5 um or more, although a high unfolding area ratio exceeding 80% is obtained, there is a problem in that the required injection pressure considerably exceeds a safe pressure of 37 kPa, making implementation impossible. Therefore, in the present invention, the thickness of the pad portion (11) is configured to be, for example, 3um~5um, thereby solving such structural problems.

Meanwhile, even in the case of the pad portion (11) having such a thickness, a more effective design can be achieved through the rib structure portion (12). For example, when the rib structure portion (12) is formed in a cross shape, the thickness of the pad portion (11) can be formed to be as thin as 1.2um.

For example, in the case of the pad portion (11) without the rib structure portion (12), when the thickness of the pad portion (11) is formed to be as thin as 1.2um, severe wrinkling occurs during injection, and when unfolded, a low unfolding area ratio of 14.77% was observed. On the other hand, when the rib structure portion (12) is formed in a cross shape, even when the thickness of the pad portion (11) is formed to be as thin as 1.2um, a high unfolding area ratio was observed even at a low injection pressure of 10 kPa due to the improved elastic restoring force. At this time, when the thickness of the rib structure portion (12) is 5um, the unfolding area ratio is 30.92%, and when the thickness is 15um, a significantly improved unfolding area ratio of 100% was observed. As a result of analysis through computational fluid dynamics (CFD) and finite element analysis (FEA), when the thickness of the pad portion (11) is formed to be as thin as 1.2um, the most optimized values satisfy the condition that when the thickness of the rib structure portion (12) is 15um~25um, the unfolding area ratio exceeds 80% at an injection pressure of less than 15 kPa.

Due to this, even when the pad portion (11) is formed to be very thin, the injection pressure can be minimized. In this case, when the rib structure portion (12) intersects in a cross shape, an angle of 90 degrees between the two rib structure portions (12) was the most suitable. When the thickness of the pad portion (11) is 1.2um and the thickness of the rib structure portion (12) is 15um, as the angle between the two rib structure portions (12) (symbol a in FIG. 4) increases, a consistent trend is observed in which the uniaxial bending stiffness increases along the X-axis (the X-axis in FIG. 4) and decreases proportionally along the Y-axis (the Y-axis in FIG. 4). Therefore, as a result, the ratio of the uniaxial bending stiffness in the X-axis direction and the Y-axis direction increases as the angle between the two rib structure portions (12) (symbol a in FIG. 4) increases. Equal bending stiffness along each axis is achieved when the angle between the two rib structure portions (12) (symbol a in FIG. 4) is 90 degrees, so that the restoring force is evenly distributed and the maximum unfolding area ratio is obtained. When the bending stiffness along the X-axis increases, the injection pressure inevitably increases and, as a result, the applied strain increases. Accordingly, when the angle between the two rib structure portions (12) (symbol a in FIG. 4) is 90 degrees, although the injection pressure is not the minimum, there is an advantage in a significantly improved unfolding area ratio due to symmetry. That is, when the angle between the two rib structure portions (12) (symbol a in FIG. 4) is 90 degrees, the highest unfolding area ratio is obtained while ensuring a safe injection pressure.

The pad portion (11) may comprise a first mesh portion (111) in which a plurality of rod shapes extending in a radial direction are spaced apart from each other in a circumferential direction, and a second mesh portion (112) formed so as to intersect with the rod shapes of the first mesh portion (111), and in which a plurality of circular shapes forming concentric circles are spaced apart from each other in a radial direction.

There are a plurality of points which are the portions where the first mesh portion (111) and the second mesh portion (112) intersect exist, and an electrode (15) or a sensor (14) may be installed at any of the plurality of intersection portions.

The second mesh portion (112) may have a support structure (13) formed on a circular shape formed at the outermost portion.

As described above, the pad portion (10) having a radial mesh structure can alleviate the pressure applied during the injection process through the syringe (S).

The rib structure portion (12) is disposed on the pad portion (11) to increase the resilience of the pad portion (11). The rib structure portion (12) may be disposed in a rod shape on an upper side of the pad portion (11), and the rod shapes may be formed so as to intersect in a cross shape. Here, the intersection point of the rod shapes may be disposed so as to be located at the center of the pad portion (11), and at least two may be disposed.

The rib structure portion (12) can provide structural stability to the pad portion (10) having the radial mesh structure, thereby enabling the pad portion (10), which is wrinkled due to injection through the syringe (S), to exhibit resilience uniformly distributed so that the pad portion (10) can be restored to its original shape when it is rapidly unfolded within a limited space of about 10 mm up to the surface of the cerebral cortex (B5).

When only one rib structure portion (12) is installed, the restoring force becomes weak when the sensor unit (10) is unfolded after being folded, and thus the sensor unit (10) may not be easily unfolded, and on the other hand, when too many rib structure portions (12) are installed, for example, about 5~10 rib structure portions are installed, a problem may occur in that the structural stiffness becomes too strong so that the sensor unit (10) cannot be folded. Therefore, in the present invention, as one embodiment, two rib structure portions (12) are disposed so as to intersect with each other so that the sensor unit (10) can be well folded and well unfolded, thereby providing appropriate structural stiffness and resilience.

The support structure (13) may be formed on a circular shape formed at the outermost portion of the second mesh portion (112), and a plurality of support structures may be formed on the circular shape so as to be spaced apart from each other in a circumferential direction.

If the support structure (13) is not formed on the circular shape formed at the outermost portion of the second mesh portion (112), the sensor unit (10) has a flexible form, and thus the frequency of folding increases, so that a problem may occur in that the sensor unit (10) is folded not only when necessary but also during normal use. Therefore, in the present invention, the support structure (13) is formed on the circular shape formed at the outermost portion of the second mesh portion (112), and preferably 3 support structures may be formed.

The sensor (14) may be formed at at least one of the portions where the first mesh portion (111) and the second mesh portion (112) intersect.

The sensor (14) may comprise a pressure sensor (141) for measuring intracranial pressure (ICP) and a temperature sensor (142) for measuring intracranial temperature (ICT). Here, the sensor (14) may preferably be a molybdenum disulfide (MoS₂)-based sensor. The sensor (14) may use atomically thin MOCVD-grown molybdenum disulfide (MoS₂) as a semiconductor sensing channel.

When the structure for measurement and stimulation (1) is introduced into the subdural space, the injection is performed through pneumatic pressure, and thus pressure may also be applied inside the subdural space. Therefore, the pressure sensor (141) monitors the intracranial pressure during the injection of the structure for measurement and stimulation (1), so that the injection can be safely performed, thereby improving the safety of the present invention.

The pressure sensor (141) may be formed of molybdenum disulfide (MoS₂) and may have a serpentine structure.

After the structure for measurement and stimulation (1) is placed in the subdural space, the temperature sensor (142) closely monitors a change in intracranial temperature according to a heat generation phenomenon that may occur due to operation of the structure for measurement and stimulation (1), thereby improving the safety of the present invention.

The temperature sensor (142) may be formed with gold (Au) electrodes in which molybdenum disulfide (MoS₂) sensing channels are interdigitated, and through this, sensitivity and detection may be greatly improved within a standard intracranial temperature range.

The electrode (15) may be formed at at least one of the portions where the first mesh portion (111) and the second mesh portion (112) intersect. Here, the electrode (15) may be formed at a portion where the rib structure portion (12) is disposed on the first mesh portion (111) among the portions where the first mesh portion (111) and the second mesh portion (112) intersect.

The electrode (15) may be formed using any one of gold, platinum-iridium alloy, graphene, carbon nanotubes, fullerene, graphite, or diamond, or a composite thereof as a material. Here, the electrode (15) may preferably be a graphene-based electrode. The electrode (15) may be composed of CVD-grown graphene, thereby allowing precise measurement of an ECoG signal by having a high signal-to-noise ratio (SNR). This is mainly due to an excellent low contact impedance interface between the graphene-based electrode (15) and the surface of the cerebral cortex (B5). In addition, the inherent mechanical flexibility of graphene ensures that the electrode (15) is not damaged. In the present invention, in order to further minimize the impedance of the electrode (15), four layers of graphene may be used in a contact region with the surface of the cerebral cortex (B5) through an interconnection composed of gold (Au).

The electrode (15) may be configured to measure signals generated in the brain or to deliver an external stimulus to the brain.

As described above, the sensor unit (10) of the present invention secures flexibility through the pad portion (11) having a radial mesh structure while securing structural stiffness through the rib structure portion (12), so that even if it is wrinkled to fit a syringe (S) having a narrow end opening of 1~2 mm, both structural integrity and electrical characteristics can be maintained, and it can be rapidly unfolded within a limited space of about 10 mm from the end of the syringe (S) to the surface of the cerebral cortex (B5) so as to be restored to its original shape.

The connection portion (20) may connect the electrical pad portion (30) and the sensor unit (10). The connection portion (20) may be configured to be connected in a long manner with the sensor unit (10) so that the electrical pad portion (30) can interface with a device such as an external reading device (not shown).

The electrical pad portion (30) may be formed for connection with the outside. The electrical pad portion (30) may be configured to interface with a device such as an external reading device (not shown).

The electrical pad portion (30) may be formed for connection to an external reading device or an integrated circuit, and for example, may be a wireless chip. The integrated circuit may include a wireless power supply device, a recording device, a stimulation device, and a communication device, and may be a package capable of improving biocompatibility.

The present invention can construct a system as the structure for measurement and stimulation (1) is connected to an external reading device, that is, an integrated circuit. This system is a system comprising the structure for measurement and stimulation.

Although in an example of the present invention, the electrical pad portion (30) is located outside the scalp as an example, it may be installed in a space between the skull and the skin. Accordingly, the integrated circuit may be installed in the space between the skull and the skin and connected to the electrical pad portion (30).

Specifically, the wireless power supply device is for supplying power to the integrated circuit, and the recording device is for recording measured brain waves, and the stimulation device is for providing stimulation to the brain, and the communication device is a component for transmitting the measured brain waves to the outside and receiving an external command.

The integrated circuit records brain-wave signals or neural signals acquired through the electrode 15 to acquire biological information and detect abnormal signals. When an abnormality is detected, the integrated circuit applies energy such as current, voltage, magnetic field, or electric field stimulation, or the like, to perform neuro-modulation so as to treat a disease or regulate brain activity.

The integrated circuit comprises a wireless chip power supply unit (not shown) which is a wireless power supply device, a recorder (not shown) which is a recording device, a stimulator (not shown) which is a stimulation device, a chip controller (not shown), and a communication device (not shown).

The wireless chip power supply unit comprises a power regulator (Power Regulation) (not shown) and a battery (not shown).

The battery may perform wireless energy supply (energy harvesting) using an RF coil or the like, and may store energy supplied in a wireless manner.

The battery may be charged wirelessly using WPT (Wireless Power Transfer) technology.

The power regulator converts AC of the battery into DC and delivers it to the chip controller.

For measurement of brain waves, analog data are converted into digital data using the recorder and the data are delivered to the chip controller, and the chip controller wirelessly transmits the data to external equipment through the communication device. The recorder varies depending on the number of brain wave measurement panels.

The recorder may perform input of multi-channel brain wave measurement data and digital signal conversion.

The chip controller may control measurement signals and stimulation signals.

The communication device is wirelessly connected to an external communication network so as to enable monitoring outside the body. The communication device may wirelessly transmit a digital signal converted by the recorder through an electrode or an antenna.

In consideration of problems such as heat generation of packaging , the integrated circuit should secure biocompatibility by allowing a package material of the integrated circuit to have compatibility with a living body, and the chip should not be affected during formation of encapsulation.

As the package material of the integrated circuit, any material that is generally used in vivo in the art may be used without limitation, and PDMS (Polydimethylsiloxane), Parylene C, Polyimide, a biocompatible UV resin, and the like, are suitable.

Specifically, when brain waves are measured through the electrode (15; graphene electrode layer) exposed to the outside from the sensor unit (10) of the structure for measurement and stimulation (1) and delivered to the integrated circuit, they may be wirelessly transmitted to the outside through the integrated circuit.

When an abnormality is detected in the brain wave signals measured by the chip controller, the abnormality is transmitted to the outside through the communication device, and a command is delivered to the stimulator so that an electrical stimulation signal is applied to the electrode (15). The stimulator varies depending on the number of electrical stimulation channels. The stimulator may generate or deliver a therapeutic stimulation commanded by the chip controller.

The brain waves measured through the electrode (15) of the sensor unit (10) of the structure for measurement and stimulation (1) may be converted into digital signals through the recorder and delivered to the integrated circuit, and they may be wirelessly transmitted to smart devices such as a smartphone or a smart pad, or to a computer. One or more recorders may be comprised.

Conversely, radio waves received from the outside through the integrated circuit may be delivered to the brain through the electrode (15) of the sensor unit (10) of the structure for measurement and stimulation (1) as electrical stimulation signal.

Through such a process, when an abnormality is detected in brain waves measured by the electrode (15) of the sensor unit (10), the abnormality is transmitted to the outside, and the integrated circuit may be controlled so as to provide an electrical signal or the like to the electrode (15) of the sensor unit (10). For example, the integrated circuit may deliver a command to the stimulator so as to stimulate the cerebral cortex. One or more stimulators may be comprised.

On the other hand, the integrated circuit may also serve to deliver external radio waves as an electrical stimulation signal to the brain without directly receiving the external radio waves, by allowing external radio waves received by a separate wireless communication device to be delivered to the integrated circuit through an electrode or an antenna as an electrical stimulation signal.

As described above, the structure for measurement and stimulation (1) according to an example of the present invention and a system comprising the same have an effect in that the rib structure portion (12) is installed on the pad portion (11) having a mesh structure to provide structural stability to the structure for measurement and stimulation (1), and when the pad portion (11) having a flexible mesh structure is folded and then unfolded during injection into the brain, resilience uniformly distributed may be exerted so that sufficient unfolding can be exhibited.

In addition, the structure for measurement and stimulation (1) according to an example of the present invention and the system comprising the same have a flexible radial mesh structure and thus have inherent mechanical flexibility, and due to this, there is an effect in that the structure is not damaged.

Through this, the present invention enables minimally invasive implantation for diagnosis and treatment of various brain diseases, and has an effect in that efficient injection and adaptive contact with the cortical surface are possible without damaging brain tissue through a mesh structure having excellent mechanical robustness and flexibility. In addition, when the graphene multi-channel electrode (15) and the molybdenum disulfide-based sensor (14) appropriately contact the surface of the cerebral cortex, seizures may be acutely detected from ECoG signals, electrotherapy for control of epileptic discharges may be performed according to a high signal-to-noise ratio (SNR) and excellent charge transfer efficiency, and intracranial pressure (ICP) and intracranial temperature (ICT) monitoring may be clearly performed.

Ultimately, through the present invention, there is an effect in that surgical risks during treatment of various brain diseases can be reduced and barriers to devices implantable in the brain can be lowered.

Although the present invention has been described in detail through specific examples, this is for the purpose of describing the present invention in detail, and the present invention is not limited thereto, and it will be apparent that modifications or improvements thereof may be made by those skilled in the art within the technical spirit of the present invention.

Simple modifications or changes of the present invention are all regarded as belonging to the scope of the present invention, and the specific scope of protection of the present invention will be made clear by the appended claims.

## Claims

1. A structure for signal measurement and electrical stimulation comprising a sensor unit that measures signals generated in a brain or delivers external stimulation to the brain,
wherein the sensor unit,
comprises a pad portion having a flexible property; and
a rib structure portion disposed on the pad portion to increase resilience of the pad portion.

2. The structure for signal measurement and stimulation according to claim 1, wherein the rib structure portion,
is disposed in rod shapes on an upper side of the pad portion, and is formed such that the rod shapes intersect with each other.

3. The structure for signal measurement and stimulation according to claim 1 or 2, wherein the pad portion,
has a radial mesh structure.

4. The structure for signal measurement and stimulation according to any of claims 1 to 3, wherein the pad portion,
comprises a first mesh portion in which a plurality of rod shapes extending in a radial direction are spaced apart from each other in a circumferential direction; and
a second mesh portion formed to intersect with the rod shapes of the first mesh portion, and in which a plurality of circular shapes forming concentric circles are spaced apart from each other in a radial direction.

5. The structure for signal measurement and stimulation according to claim 4, wherein an electrode or a sensor is installed at portions where the first mesh portion and the second mesh portion intersect.

6. The structure for signal measurement and stimulation according to claim 4 or 5, wherein in the second mesh portion,
a support structure is formed on a circular shape formed at the outermost portion, and the support structure is formed in plural and spaced apart from each other in a circumferential direction on the circular shape.

7. The structure for signal measurement and stimulation according to claim 5, wherein the electrode is,
composed of any one among gold, platinum-iridium alloy, graphene, carbon nanotubes, fullerene, graphite or diamond, or a composite thereof.

8. The structure for signal measurement and stimulation according to claim 5 or 7, wherein the electrode is,
installed at a portion at which the rib structure portion is disposed on the first mesh portion.

9. The structure for signal measurement and stimulation according to claim 5 or 7 or 8, wherein the electrode, measures signals generated in a brain or delivers external stimulation to the brain, and
the sensor comprises a pressure sensor measuring intracranial pressure (ICP) or a temperature sensor measuring intracranial temperature (ICT).

10. The structure for signal measurement and stimulation according to any one claim of claims 1 to 9, further comprising an electrical pad portion for external connection; and
a connection portion connecting the electrical pad portion and the sensor unit.
